(19) Europäisches Patentamt
European Patent Office
Office européen des brevets

(11) Publication number: 0 048 025
B1

(12) # EUROPEAN PATENT SPECIFICATION

(45) Date of publication of patent specification: 15.01.86

(21) Application number: 81107302.2

(22) Date of filing: 16.09.81

(51) Int. Cl.[4]: C 07 C 103/737,
C 07 C 149/243,
C 07 C 123/00, C 07 C 129/12,
A 61 K 31/40 // C07D339/08,
C07C69/716, C07D487/04,
C07C119/18

(54) **Antibacterial composition of thienamycin type compound and a dipeptidase inhibitor.**

(30) Priority: 17.09.80 US 188178
17.09.80 US 187929

(43) Date of publication of application:
24.03.82 Bulletin 82/12

(45) Publication of the grant of the patent:
15.01.86 Bulletin 86/03

(84) Designated Contracting States:
AT BE CH DE FR GB IT LI LU NL SE

(56) References cited:
EP-A-0 007 614
EP-A-0 010 573
EP-A-0 028 778

(73) Proprietor: MERCK & CO. INC.
126, East Lincoln Avenue P.O. Box 2000
Rahway New Jersey 07065 (US)

(72) Inventor: Kropp, Helmut
18 Boyd Terrace
Kenilworth, New Jersey 07033 (US)
Inventor: Kahan, Frederick M.
2022 Brookside Drive
Scotch Plains, New Jersey 07076 (US)
Inventor: Graham, Donald W.
294 Old Tote Road
Mountainside, New Jersey 07092 (US)
Inventor: Rogers, Edward F.
61 Kings Highway
Middletown, New Jersey 07748 (US)

(74) Representative: Abitz, Walter, Dr.-Ing. et al
Abitz, Morf, Gritschneder P.O. Box 86 01 09
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

A new class of fused ring β-lactam antibiotics, including thienamycin and its semisynthetic derivatives, epithienamycins, and olivanic acids, has recently been described. These compounds which will be defined more extensively below, are hereinafter referred to as the "thienamycin class of compounds". These compounds have a high level of antibacterial activity, but are subject to extensive metabolism by mammalian species.

The kidney was identified as the primary site of metabolism, and an enzyme was purified from renal extracts which catalyzed the inactivation of thienamycin by hydrolysis of the β-lactam. By such criteria as cytological localization, substrate specificity and susceptibility to enzyme inhibitors, this enzyme is very similar if not identical to a widely studied renal dipeptidase (E.C.3.4.13.11), also referred to in the literature as "dehydropeptidase-I". However, the β-lactamase activity is exhibited only toward the thienamycin class of compounds. Indeed, there exists no precedent example of the mammalian metabolism via β-lactam cleavage of any representative of the classical β-lactam antibiotics, the penicillins and cephalosporins.

EP—A—0 007 614 discloses an antibacterial composition of thienamycin-type compound and a dipeptidase inhibitor, e.g. Z - 2 - (2,2 - dimethylcyclopropane - carboxamido) - 2 - octenoic acid.

This invention is directed to an antibacterial composition comprising 6- and (optionally) 2- substituted pen-2-em-3-carboxylic acids, 1-carbadethiapen-2-em-3-carboxylic acids or 1-aza-bicyclo [3.2.0] hept-2-ene-7-one-2-carboxylic acids and Z - 7 - (L - 2 - amino - 2 - carboxyethylthio) - 2 - (2,2 - dimethylcyclopropane - carboxamido) - 2 - heptenoic acid or its sodium, potassium, calcium or magnesium salt.

Z-7-(L-2-amino-2-carboxyethylthio)-2-(2,2-dimethylcyclopropanecarboxamido)-2-heptenoic acid and its sodium, potassium, calcium and magnesium salts selectively inhibit the metabolism of the dipeptidase [E.C.3.4.13.11] and are called "dipeptidase inhibitors".

The Z configuration (J. E. Blackwood et al., *J. Am. Chem. Soc.*, *90*, p. 509 (1968)) is assigned to the above inhibitor compounds on the basis of their NMR spectra by analogy with the work of A. Srinavasan et al. [Tetrahedron Lett., 891 (1976)].

Those dipeptidase inhibitors are used in combination with certain antibacterial compounds which are subject to renal degradation. Those antibiotics are the "thienamycin class of compounds". These compounds are classed as 6- and (optionally) 2-substituted pen-2-em-3-carboxylic acids and 1-carbadethiapen-2-em-3-carboxylic acids or 1-azabicyclo [3.2.0] hept-2-ene-7-one-2-carboxylic acids.

Specific compounds particularly useful in this invention are represented structurally in the following formula I:

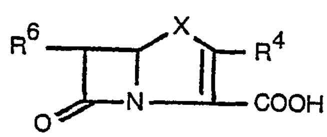

I

wherein X can be $CH_2$ or S; $R^4$ can be hydrogen; $—S—CH_2CH_2NHR^5$, wherein $R^5$ is hydrogen, acetyl, formimidoyl, acetimidoyl; $—S(O)—CH{=}CHNHCOCH_3$ and $—S—CH{=}CHNHCOCH_3$; and $R^6$ is

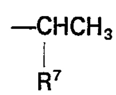

wherein $R^7$ is hydrogen, hydroxy or hydroxy-sulfonyloxy, or $R^6$ is H. All possible stereoisomeric forms are included within the above structural definition.

All of these compounds within Formula II are described in the literature. When X is $CH_2$, and $R^4$ is $SCH_2CH_2NH_2$, and $R^6$ is $CH(OH)CH_3$, the compound is known as thienamycin, an antibiotic produced by fermentation of *S. cattleya*, deposited as NRRL 8057 at Northern Regional Research Laboratories, U.S. Department of Agriculture, Peoria, Illinois, U.S.A., on November 18, 1974 described in U.S. Patent 3,950,357, issued April 13, 1976. The fermentation product N-acetyl thienamycin ($R^6$ is $CH(OH)CH_3$, and $R^5$ is acetyl), also called 924A, is described in Belgian Patent No. 848,346, issued May 16, 1977. The N-imidoyl derivatives are covered in Belgian Patent No. 848,545, issued May 20, 1977. The unsaturated side chain-containing compound, also called N-acetyl-dehydrothienamycin or $924A_5$ is a fermentation product of S. cattleya, NRRL 8057, described in Belgian Patent No. 866,035, issued October 17, 1978. Epimeric forms of N-acetyl thienamycin, also called 890A, and $890A_3$, as well as the desacetyl 890A, and desacetyl $890A_3$ are disclosed respectively in published French Patent 7,634,887, granted April 14, 1980, and Belgian Patent 848,349, issued May 16, 1977. Epimeric forms of the unsaturated thienamycin, also called $890A_2$ and $890A_5$, are described in published French Patent 7,711,891, granted April 28, 1977. The 6-hydroxy-sulfonyloxyethyl containing N-acetyl compounds, also called $890A_9$ or $890A_{10}$, are disclosed respectively, in published French Patent 7,734,456, granted June 23, 1980, and published French Patent 7,734,457, granted March 3, 1980. Desacetyl analogues of $890A_9$ and $890A_{10}$ are disclosed in DE—OS 28 05 701, French Patent 7,803,666, granted May 5, 1980. DE—OS 28 05 724, and also in French Appln. 7,803,667, filed February 9, 1978. Some

of these latter compounds in the $890A_9$ and $890A_{10}$ series are also known as derivatives of olivanic acid (see Corbett et al., *J. Chem. Soc. Chem. Commun.* 1977, No. 24, pp. 953—54). Compounds of the Formula I above when $R^4$ is hydrogen, also called descysteaminyl thienamycins, are disclosed in Belgian Patent 867,227, issued November 20, 1978.

When $R^6$ is hydrogen, and X is $CH_2$, these compounds are disclosed in German Off. 2,751,624.1, filed November 18, 1977.

A thienamycin-type antibiotic in which $R^4$ is $—SCH_2CH_2NHAc$ and $R^6$ is $C_2H_5$, has been named PS-5 and is reported by K. Okaimura et al., *J. Antibiotics 31* p. 480 (1978), see also Belgian Patent 865,578.

The compounds in which X is S, also called "penems", are described by R. B. Woodward in "Recent Advances in the Chemistry of β-Lactam Antibiotics", J. Elks (Ed), The Chemical Society, London, 1977, p. 167; R. B. Woodward, Abstracts of Uppsala University 500 Years Symposium on Current Topics in Drug Research, Uppsala, Sweden, October 1921, 1977. *Acta. Pharm. Suecica*, Vol. 14, Supplement, p. 23, and U.S. Patent 4,070,477, issued January 24, 1978.

Particularly preferred members within the thienamycin class of compounds are thienamycin the N-formimidoyl and N-acetamidoyl derivatives of thienamycin. The crystalline form of N-formimidoyl thienamycin, which has recently been described, is also useful in the practice of this invention. An example illustrating a preferred way of making this compound follows:

Illustrative Example

N-Formimidoyl thienamycin, (NFT) crystalline

Step A. Benzylformimidate hydrochloride

A 3 l. three-necked flask fitted with an addition funnel, overhead stirrer, and a reflux condenser, was charged with a mixture of benzyl alcohol (125 g., 1.15 mol) formamide (51 g., 1.12 mol) and anhydrous ether (1200 ml.). The mixture was stirred vigorously at room temperature (20—25°C) under a nitrogen atmosphere and benzoyl chloride (157 g., 1.12 mol) in 50 ml. of anhydrous ether was added dropwise using the addition funnel. The addition required approximately 50 minutes.

The reaction mixture was stirred an additional 60 minutes at room temperature. The ether was removed by decantation and 300 ml. of acetic anhydride in 500 ml. of anhydrous ether was added. The mixture was stirred 30 minutes at room temperature. The precipitate was allowed to settle and the ether-acetic anhydride was again removed by decantation. The solid was collected by filtration, washed with 500 ml. of ether and dried *in vacuo* over KOH at 25°C for 2 hrs. to give 130 g. (67%) of benzylformimidate hydrochloride as a white solid.

The product was assayed by NMR δ (DMSO) 5.7 (s, 2H, $\phi CH_2$), 7.5 (s, 5H, $\phi$), 9.0 (s, 1H, HC=N). The product is thermally unstable. It decomposes to formamide and benzyl chloride at 0°C and above. However, no appreciable decomposition was detected on storage at −20°C for 2 months.

Step. B. Derivatization of thienamycin

Thienamycin (in the form of a 6 l. aqueous solution, pH=6.5, concentrate from the fermentation broth, containing 28 g. thienamycin) was placed in a large beaker (12 l) and cooled at 0°C. The beaker was equipped with a pH meter and an efficient high speed stirrer. The pH was raised to 8.5 by the careful addition of 3N KOH (KOH was added dropwise via syringe to the stirred solution). The solution was treated with 6 equivalents of solid benzyl formimidate hydrochloride (~100 g.) in portions while maintaining the pH at 8.5±0.3 by the addition of 3N KOH (200 ml.) using a syringe. The addition required 3—5 min. The reaction mixture was stirred for 6 min. at 0°C and then assayed by liquid chromatography to insure completion of the reaction. The solution was adjusted to pH 7 with 1N HCl. The volume of the reaction mixture was measured, and the solution was assayed by UV. The neutralized reaction mixture was concentrated to 15 g./l. on the reverse osmosis unit at <10°C. The volume of the concentrate was measured and the pH was adjusted to 7.2—7.4, if necessary. The concentrate was filtered through a medium porosity sintered glass funnel to remove any solids present after concentration.

Step C. Dowex 50W×2 chromatography

The concentrate (750—1000 ml., 15—20 g.) was applied to 0°C. to a precooled 18 l. column of Dowex 50W×2 in the potassium cycle (200—400 mesh resin) and the column was eluted at 0—5°C with distilled deionized water a flow rate of 90 ml/min. and a head pressure of 0—45 psig.

Forerun fractions of 4 l., 2 l., and one l., were collected followed by 18 fractions of 450 ml. each, and one final fraction of 2 l. Each fraction was assayed by UV (1/100 dilution, $NH_2OH$ extinction was omitted) and the total amount of NFT present in each fraction was calculated. The beginning and end fractions were assayed for liquid chromatography purity and the desired rich cut fractions were combined. The pH of the combined rich cuts was determined by both pH meter and bromothymol blue indicating solutions and was adjusted to pH 7.2—7.4 if necessary. The combined rich cuts (3—4 l.) were then assayed by UV and the total formamidine content was determined, 15—16 g., 75% yield from the column. The rich cuts were concentrated on the reverse osmosis unit at <10°C as far as possible, then the concentration to 33 g./l. was completed on the circulatory evaporator at less than 28°C. A total volume of about 500 ml. concentrate was obtained.

Step D. Crystallization of N-formimidoyl thienamycin

The concentrate from the previous step is adjusted to pH 7.3, if necessary and N-formimidoyl thienamycin content assayed by UV, was about 85—90%. The concentrate was filtered through a sintered glass funnel (medium porosity) into a large Erlenmeyer flask. Five volumes (~2200 ml.) of 3A ethanol was filtered into the concentrate and the solution was stirred at room temperature for 10 minutes and at 0°C for 12—24 hrs.

The crystals were filtered by suction filtration and washed with 0.1 volume (~250 ml.) of 0°C 80% 3A ethanol followed by 1/25 volume (100 ml.) of 3A ethanol at room temperature. The crystals were dried *in vacuo* for 12—24 hrs. to give approximately a 40% overall yield of N-formimidoyl thienamycin (10—12 g.).

Analytical results on a 50 g. blend of N-formimidoyl thienamycin, prepared as above, are as follows:

C, theory 45.42%; found, 45.82%
H, theory 6.03%; found, 5.72%
N, theory 13.24%; found, 13.10%
S, theory 10.10%; found, 10.14%

residue on ignition, predicted 0.5, found 0.47%; $[\alpha]_D^{25}$=89.4°, T.G.=6.8%, UVλmax 300 μm, E%=328.

The composition of this invention can be in the form of a pharmaceutical composition containing the two compounds in a pharmaceutically acceptable carrier. The two compounds are preferably employed in amounts so that the weight ratio of the thienamycin class compound to inhibitor is 1:3 to 30:1, and more preferably 1:1 to 5:1.

The most preferred dosage regimen and level is the combination of crystalline N-formimidoyl thienamycin and the other being the crystalline form of Z - 7 - (L - 2 - amino - 2 - carboxyethylthio) - 2 - (2,2 - dimethylcyclopropanecarboxamido) - 2 - heptenoic acid, co-administered in a sterile aqueous IV injection form (sodium salt), at a level of 250 or 500 mg of the thienamycin compound and about 1:1 (weight) of the heptenoic acid, or 250 or 500 mg. This dose is given to humans (each assumed to weigh about 80 kg.) from 1 to 4 times daily, or 3.1—25 mg/kg/day of each drug.

The components are employed in pharmaceutically acceptable carriers such as conventional vehicles adapted for oral administration such as capsules, tablets, or liquid solutions or suspensions. The components can also be dissolved in a vehicle adapted for administration by injection. Suitable formulations for oral use, may include diluents, granulating agents, preservatives, binders, flavoring agents, and coating agents. An example of an oral use composition is the combination of active ingredients, intermixed in the dry pulverulent state with gelatin, starch, magnesium stearate, and alginic acid, and pressed into a tablet.

As noted, disposition studies with thienamycin, its natural analogs and its semi-synthetic derivatives have revealed a major metabolic degradation pathway of elimination in the various species examined (mouse, rat, dog, chimpanzee, Rhesus monkey). The extent of metabolism is reflected in low urinary recovery and short plasma half-lives. The nature of this degradation was demonstrated to be lactam cleavage by the renal dipeptidase (E.C.3.4.13.11), described first by Bergmann, M. and Schleich, H., *Z. Physiol. Chem., 205* 65 (1932); see also Greenstein, J. P., *Advances in Enzymology*, Vol. VIII, Wiley-Interscience, (1948), New York, and Campbell, B. J.; Lin, Y—C., Davis, R. V. and Ballew, E., "The Purification and Properties of Particulate Renal Dipeptidase", *Biochim. Biophys. Acta., 118*, 371 (1966).

In order to demonstrate the ability of the dipeptidase inhibitor to suppress the action of the renal dipeptidase enzyme, an *in vitro* screen procedure was followed. This measured the ability of compounds to inhibit hydrolysis of glycyldehydrophenylalanine (GDP) by a solubilized preparation of dipeptidase isolated from hog kidneys. The procedure is as follows: to a 1 ml. system containing 50 mM "MOPS" (3-(N-morpholino) propanesulfonic acid) buffer, pH 7.1, is added 5 μg of lyophilized enzyme, and the test compound at a final concentration of 0.1 mM. After a five minute incubation at 37°C, GDP is added to a final concentration of 0.05 mM. Incubation is continued for 10 minutes, at 37°C and hydrolysis of GDP is measured by the change in optical density with time at 275 nm. Inhibition of the enzyme is gauged by comparison to a standard run containing no inhibitor and is expressed as the inhibitor binding constant, $K_i$. This is the concentration of the inhibitor which achieves 50% inhibition of enzyme.

The substrate GDP is employed in preference to thienamycin in this screen because it has a much higher maximal velocity of hydrolysis by renal dipeptidase, thereby reducing the amount of enzyme required. Both GDP and thienamycin have a similar affinity for renal dipeptidase; furthermore, $K_i$'s of inhibitors tested have been identical for the two substrates.

In vitro test data

A 1 ml. system of 50 mM "MOPS" buffer, pH 7.1, is used. To this is added 5 μg of the pig renal enzyme and an amount of Z - 7 - (L - 2 - amino - 2 - carboxyethylthio) - 2 - (2,2 - dimethylcyclopropane - carboxamido) - 2 - heptenoic acid to bring its final concentration to 0.1 mM. After a five minute incubation at 37°C, an amount of GDP is added to bring its final concentration to 0.05 mM. The system is again incubated for 10 minutes, at 37°C. Hydrolysis of GDP is measured by its change in optical density with time at 275 nm. Inhibition of the enzyme is gauged by comparison to a standard run containing no inhibitor and is presented as percent inhibition. The $K_i$ is a constant indicating the concentration of inhibitor necessary to

produce 50% inhibition of enzyme. It is a calculated value obtained from running multiple *in vitro* assays, as above, at concentrations resulting in inhibition below and above the 50% inhibition point. The $K_i$ of the inhibitor was 0.21 μM.

Preparation of 7-(L-2-amino-2-carboxyethylthio)-2-(2,2-dimethylcyclopropanecarboxamido)-2-heptenoic acid

Z-7-bromo-2-(2,2-dimethylcyclopropanecarboxamido)-2-heptenoic acid (185 mg, 1.05 mmoles) is dissolved in 2.02 ml NAOH solution (2.0 N), and deoxygenated by bubbling a stream of nitrogen gas through it for a minute. Then cysteine. HCl (185 mg, 1.05 mmoles) is added all at once and the reaction stirred at room temperature in a $N_2$ atmosphere for 3 hours. The reaction mixture is applied to 2×20 cm column of Dowex 50×4 (100—200 mes $H^+$), and eluted with 300 ml $H_2O$), then 200 ml of 2N $NH_3$ solution. Ammonia evaporated under reduced pressure to give 284 mg of a yellowish glass. This product is dissolved in 4 ml ethanol, and the insoluble material filtered. The filtrate is added dropwise to rapidly stirred diethylether (150 ml). The solid which precipitates is filtered, washed with ether and dried to yield 171 mg product, having one spot (ninhydrin positive) in TLC (nBuOH, HOAc, $H_2O$; 4:1:1) rf. about 6; NMR is consistent with the desired structure.

| Anal. ($C_{16}H_{26}N_2O_5S$) | Calcd. | Found |
|---|---|---|
| C | 53.61 | 52.55 |
| H | 7.31 | 7.40 |
| N | 7.81 | 7.89 |
| S | 8.94 | 9.63 |

Z-7-bromo-2-(2,2-dimethylcyclopropanecarboxamido)-2-heptenoic acid has been prepared in the following way:

Reaction of 1,5-dibromoheptane with alkyl 1,3-dithiane-2-carboxylate to the alkylated dithiane which is further reacted with N-bromosuccinimide to the corresponding ethyl 7-bromo-2-keto-heptanoate. The free acid thereof is reacted with 2,2-dimethylcyclopropanecarboxamide.

Preparation of sodium Z-7-(L-2-amino-2-carboxethylthio)-2-(2,2-dimethylcyclopropane-carboxamido)-2-heptenoic acid

A. Grignard preparation of ethyl-7-chloro-2-oxoheptanoate

Equimolar amounts (8 moles each) of 1-bromo-5-chloropentane and magnesium are reacted in tetrahydrofuran (960 ml) at 25°C. The flask is charged with mg. in the THF and the bromochloropentane added over 1 hr, then aged 2 hrs. After the reaction was judged complete, the reaction solution was added (cooled to −15°C) to 16 moles of diethyloxalate in 1856 ml tetrahydrofuran, while maintaining the temperature at −10°C. 3N · HCl was added to quench, keeping the temperature below 25°C. After stripping solvents, the calculated yield is 48.8% of the ethyl-7-chloro-2-oxoheptanoate.

B. Condensation and hydrolysis

S-2,2-dimethylcyclopropyl carboxamide (1017 g), 2143.6 g of ethyl-7-chloro-2-ketoheptanoate, 9 liters of toluene and 12 g of p-toluene sulfonic acid were charged to a 22 L. flask, and heated to reflux with stirring. After 23 hrs., liquid chromatography showed the expected product ratio, and 4 L. of toluene were removed under slightly reduced pressure. The pot was charged with water, neutralized to pH 7 with 2N NaOH, and vacuum distilled leaving a final pot volume of about 5 liters.

This was hydrolyzed by adding 1760 g of 50% aq. NaOH (4 liters water) and stirring overnight. The flask was charged with 4 L. methylene chloride, and pH adjusted to 8.8 using HCl. unreacted amide crystallized out. The organic layers were separated from water, and then evaporated. The gummy residue was dissolved in 8 liters water containing 720 g 50% NaOH, and to this solution was charged 1818 g L. cysteine HCl · $H_2O$, 2 kg ice, 2484 g 50% NaOH and 1 liter water.

The pH of this solution, after aging overnight at room temperature, is adjusted to 3.0 with conc. HCl, and the resulting gummy suspension heated to 95°C to afford a clear solution. After 30 minutes, no E isomer could be detected by lc. After work-up and purification, the overall yield was 50%. This material was recrystallized from acetonitrile. 1500 g of the recrystallized material was dissolved in 6 liters water and 910 ml 3.88 N NaOH, then neutralized to pH 7, and lyophilized to afford 1569 g (98.6%) of the title compound: Analysis: calcd: C, 50.52; H, 6.62; N, 7.36; S, 8.43; Na, 6.04; found: C, 50.71; H, 6.78; N, 7.49; S, 8.52; Na, 5.92.

**Claims for the Contracting States BE CH DE FR GB IT LI LU NL SE:**

1. An antibacterial composition comprising 6- and (optionally) 2-substituted pen - 2 - em - 3 -

carboxylic acids, 1 - carbadethia - pen - 2 - em - 3 - carboxylic acids or 1 - aza - bicyclo [3.2.0] hept - 2 - ene - 7 - one - 2 - carboxylic acids and Z - 7 - (L - 2 - amino - 2 - carboxyethylthio) - 2 - (2,2 - di - methylcyclopropane - carboxamido) - 2 - heptenoic acid or its sodium, potassium, calcium or magnesium salt.

2. The antibacterial composition of claim 1 comprising N - formimidoyl thienamycin and Z - 7 - (L - 2 - amino - 2 - carboxyethylthio) - 2 - (2,2 - dimethylcyclopropane - carboxamido) - 2 - heptenoic acid or its sodium, potassium, magnesium or calcium salt.

**Claims for the Contracting State AT:**

1. Process for preparing an antibacterial composition comprising mixing 6- and (optionally) 2-substituted pen - 2 - em - 3 - carboxylic acids, 1 - carbadethia - pen - 2 - em - 3 - carboxylic acids or 1 - aza - bicyclo [3.2.0] hept - 2 - ene - 7 - one - 2 - carboxylic acids and Z - 7 - (L - 2 - amino - 2 - carboxyethylthio) - 2 - (2,2 - dimethylcyclopropane - carboxamido) - 2 - heptenoic acid or its sodium, potassium, calcium or magnesium salt.

2. Process of claim 1 comprising mixing N-formimidoyl thienamycin and Z - 7 - (L - 2 - amino - 2 - carboxyethylthio) - 2 - (2,2 - dimethylcyclopropane - carboxamido) - 2 - heptenoic acid or its sodium, potassium, magnesium or calcium salt.

**Patentansprüche für die Vertragsstaaten BE CH DE FR GB IT LI LU NL SE:**

1. Eine antibakterielle Zusammensetzung, enthaltend 6- und (gegebenenfalls) 2-substituierte Pen - 2 - em - 3 - carbonsäuren, 1 - Carbadethia - pen - 2 - em - 3 - carbonsäuren oder 1 - Azabicyclo[3.2.0]hept - 2 - en - 7 - on - 2 - carbonsäuren und Z - 7 - (L - 2 - Amino - 2 - carboxyethylthio) - 2 - (2,2 - dimethylcyclopropan - carboxamido) - 2 - heptensäure oder deren Natrium-, Kalium-, Calcium- oder Magnesiumselz.

2. Die antibakterielle Zusammensetzung des Anspruchs 1, enthaltend N-Formimidoylthienamycin und Z - 7 - (L - 2 - Amino - 2 - carboxyethylthio) - 2 - (2,2 - dimethylcyclopropancarboxamido) - 2 - heptensäure oder deren Natrium-, Kalium-, Magnesium- oder Calciumsalz.

**Patentansprüche für den Vertragsstaat AT:**

1. Verfahren zur Herstellung einer antibakteriellen Zusammensetzung, umfassend das Vermischen von 6- und (gegebenenfalls) 2 - substituierten Pen - 2 - em - 3 - carbonsäuren, 1 - Carbadethia - pen - 2 - em - 3 - carbonsäuren oder 1 - Azabicyclo[3.2.0]hept - 2 - en - 7 - on - 2 - carbonsäuren und Z - 7 - (L - 2 - Amino - 2 - carboxyethylthio) - 2 - (2,2 - dimethylcyclopropan - carboxamido) - 2 - heptensäure oder deren Natrium-, Kalium-, Calcium- oder Magnesiumsalz.

2. Verfahren nach Anspruch 1, umfassend das Vermischen von N-Formimidoylthienamycin und Z - 7 - (L - 2 - Amino - 2 - carboxyethylthio) - 2 - (2,2 - dimethylcyclopropan - carboxamido) - 2 - heptensäure oder deren Natrium-, Kalium-, Magnesium- oder Calciumsalz.

**Revendications pour les Etats Contractants BE CH DE FR GB IT LI LU NL SE:**

1. Une composition antibactérienne comprenant des acides pén - 2 - ème - 3 - carboxyliques, des acides 1 - carbadéthia - pén - 2 - ème - 3 - carboxyliques ou des acides 1 - aza - bicyclo[3.2.0]hept - 2 - ène - 7 - one - 2 - carboxyliques 6- et (éventuellement) 2-substitués et l'acide Z - 7 - (L - 2 - amino - 2 - carboxyéthylthio) - 2 - (2,2 - diméthylcyclopropane - carboxamido) - 2 - hepténoïque ou son sel de sodium, de potassium, de calcium ou de magnésium.

2. La composition antibactérienne de la revendication 1 comprenant de la N-formimidoyl thiénamycine et de l'acide Z - 7 - (L - 2 - amino - 2 - carboxyéthylthio) - 2 - (2,2 - diméthylcyclopropane - carboxamido) - 2 - hepténoïque ou son sel de sodium, de potassium, de magnésium ou de calcium.

**Revendications pour l'Etat Contractant AT:**

1. Procédé de préparation d'une composition antibactérienne comprenant le mélange d'acides pén - 2 - ème - 3 - carboxyliques d'acides 1 - carbadéthia - pén - 2 - ème - 3 - carboxyliques ou d'acides 1 - aza - bicyclo[3.2.0]hept - 2 - ène - 7 - one - 2 - carboxyliques 6- et (éventuellement) 2-substitués et d'acide Z - 7 - (L - 2 - amino - 2 - carboxyéthylthio) - 2 - (2,2 - diméthylcyclopropane - carboxamido) - 2 - hepténoïque ou son sel de sodium, de potassium, de calcium ou de magnésium.

2. Procédé selon la revendication 1, comprenant le mélange de N-formimidoyl thiénamycine et d'acide Z - 7 - (L - 2 - amino - 2 - carboxyéthylthio) - 2 - (2,2 - diméthylcyclopropane - carboxamido) - 2 - hepténoïque ou de son sel de sodium, de potassium, de magnésium ou de calcium.